# EUROPÄISCHE PATENTSCHRIFT

(11) **EP 0 981 293 B1**
(45) Veröffentlichungstag und Bekanntmachung des Hinweises auf die Patenterteilung: **16.10.2002**
(21) Anmeldenummer: 98935012.9
(22) Anmeldetag: 01.07.1998
(51) Int. Cl.: A61B 3/16, A61B 5/00

(54) **VORRICHTUNG ZUR MESSUNG DES AUGENINNENDRUCKS**
DEVICE FOR MEASURING THE INTRA-OCULAR PRESSURE
INSTRUMENT A MESURER LA PRESSION INTRA-OCULAIRE

(30) Priorität: 01.07.1997 DE 19728069
(43) Veröffentlichungstag der Anmeldung: 01.03.2000
(73) Patentinhaber: Acritec GbmH, 16548 Glienicke (DE); Mesotec Gesellschaft für Medizinische Sensortechnik mbH, 30165 Hannover (DE)
(72) Erfinder: SCHNAKENBERG, Uwe, D-52074 Aachen (DE); MOKWA, Wilfried, D-47800 Krefeld (DE); KREINER, Christine, D-81545 München (DE); RICHTER, Horst, D-52146 Würselen (DE)
(74) Vertreter: Nöth, Heinz, Dipl.-Phys.
(86) Internationale Anmeldenummer: EP9804072
(87) Internationale Veröffentlichungsnummer: WO99001063

(56) Entgegenhaltungen:
- DE-A- 4 341 903
- US-A- 5 005 577

## Beschreibung

Die Erfindung betrifft eine Vorrichtung nach dem Oberbegriff des Patentanspruches 1. Eine derartige Vorrichtung ist aus der US 5 005 577 bekannt.

Messungen des Augeninnendrucks (Glaukom) werden im Normalfall während eines Besuches beim Augenarzt routinemäßig durchgeführt. Bei Patienten mit bekanntem Glaukom werden die Druckmessungen in regelmäßigen Abständen von sechs bis zwölf Wochen durchgeführt. Diese singulären Druckmessungen sind jedoch wenig aussagekräftig, da der intraokulare Druck tageszeitlichen Schwankungen unterliegt. Um eine objektive Aussage darüber zu erhalten, ob eine pathologische Drucksituation vorliegt, müssen kontinuierliche Druckmessungen über einen längeren Zeitraum durchgeführt werden, anhand deren dann erst entschieden werden kann, ob und welche Therapie einzuleiten ist. Dies ist mit den derzeit zur Verfügung stehenden Geräten zur Messung des intraokularen Druckes (Tonometer) nicht möglich.

Die zur Verfügung stehenden Tonometer (Applanations- und Impressionstonometer) ermöglichen eine genaue Bestimmung des intraokularen Druckes (Grehn F., Leydhecker W., "Augenheilkunde" 26. Auflage, Springer-Verlag, Seiten 244, 245.). Ihre Praxistauglichkeit bei der routinemäßigen Früherkennung ist jedoch eingeschränkt. Mit den bekannten Meßgeräten wird bei der Messung die schmerzempfindliche Hornhaut des Auges berührt, so daß die Druckmessung nur nach lokaler Anästhesie des Auges durchgeführt werden kann. Die bekannten Meßgeräte liefern unbrauchbare Werte bei abnormer Hornhautoberfläche durch Ödeme oder Narben sowie bei starkem Astigmatismus. Untersuchungen im Anschluß an Augenoperationen, um den Erfolg der Operation zu überwachen, sind nicht möglich. Non-contact-Tonometer, die derzeit am Markt sind, erreichen nicht die Meßgenauigkeit, die für eine sichere Diagnose erforderlich sind. Insbesondere im hohen Augendruckbereich, der irreversible Schädigungen der Sehnerven zur Folge hat, sind die Messungen mit den derzeit vorhandenen Non-contact-Tonometern zu ungenau und unzuverlässig. Da auch bei diesen Tonometern die Applanation der Hornhaut zur Messung benutzt wird, sind die mit dieser Applanation verbundenen Nachteile vorhanden.

Die eingangsgenannte Vorrichtung, welche aus der US 5 005 577 bekannt ist, beinhaltet eine in ein Auge mittels einer Intraokularlinse implantierbare Fernmeßeinrichtung, welche einen Durcksensor, eine die Sensorsignale in drahtlos übertragbare Informationen wandelnde Einrichtung und eine Sendeeinrichtung enthält. Mittels einer außerhalb des Auges angeordneten Empfangseinrichtung können die von der implantierten Sendeeinrichtung gesendeten Informationen empfangen werden und in Daten des Augeninnendrucks gewandelt werden, die aufgezeichnet werden können. Ferner kann die bekannte Vorrichtung eine von außen gespeiste Energiequelle, beispielsweise ein fotoelektrisches Element aufweisen, um einen aktiven Sensor und Telemetriesender für die Datenübertragung zu erreichen.

Um objektiv beurteilen zu können, ob eine pathologische Drucksituation im Auge vorliegt, müssen Druckmessungen ausgewertet werden, die über einen längeren Zeitraum hin sich erstrecken. Die Erfassung dieser länger andauernden Druckmessungen ist mit den bekannten Geräten aufwendig.

Aufgabe der Erfindung ist es daher, eine Vorrichtung zur Messung des Augeninnendrukkes zu schaffen, mit welcher ein erhöhter Augeninnendruck (Glaukom) kontinuierlich überwacht und bedarfsweise abgerufen werden kann.

Diese Aufgabe wird erfindungsgemäß durch das Kennzeichen des Patentanspruches 1 gelöst.

Durch den Datalogger, der in der implantierten Fernmeßeinrichtung vorhanden ist, erreicht man eine kontinuierliche Aufzeichnung und Speicherung der Meßwerte über einen langen Zeitraum. Diese können bedarfsweise innerhalb eines kurzen Zeitraums, beispielsweise innerhalb von Sekunden bis Minuten abgerufen werden. Nur während dieses Zeitraums muß ein Hilfsgerät, beispielsweise in Form eines Handgerätes, einer Brille oder einer Augenbinde, für den Empfang der Meßdaten zum Einsatz gebracht werden.

Die Fernmeßeinrichtung, welche in bevorzugter Weise als aktive Telemetrie ausgebildet ist, kann an einer geeigneten Stelle, beispielsweise Sulkus Kapselsack, Vorderkammer, als einsetzbares Implantat angeordnet sein. Dieses Implantat kann als Intraokularlinse ausgebildet sein, wobei die Fernmeßeinrichtung außerhalb des optischen Linsenteils bevorzugt an einem das Linsenteil umgebenden Haptikrand vorgesehen ist. Hierdurch. wird eine intelligente Linse zur multifunktionalen Meßwerterfassung durch aktive Telemetrie und Intregation des Dataloggers ermöglicht, mit welcher Meßwerte gespeichert werden können.

Als Sensoren eignen sich solche, mit denen der Augeninnendruck erfaßt werden kann. Insbesondere ist der Drucksensor bzw. sind die Drucksensoren im Gegensatz zu bekannten kapazitiv messenden Sensoren (Sensors and Actuators A, 37 - 38 (1993) 93 - 105) in Oberflächenmikromechanik ausgebildet. Zusätzlich können Elektroden zur Stimulation und Ableitung von Reizpotentialen vorgesehen sein. Der Drucksensor, die zugehörige Signalverarbeitungsschaltung, der Datalogger und die sensorseitigen telemetrischen Komponenten, insbesondere Spule und Kondensatoren, sind in bevorzugter Weise monolithisch in einem Chip, beispielsweise Silicium- bzw. Siliconchip integriert. Durch induktive Signal- und Energieübertragung zwischen der implantierten Fernmeßeinrichtung und der außerhalb des Auges vorgesehenen Empfangseinrichtung erreicht man eine aktive Telemetrie, wobei die Energieversorgung der im Auge angeordneten Fernmeßeinrichtung durch induktive Energieübertragung erreicht werden kann. Hierzu besitzen sowohl die implantierte Fernmeßeinrichtung als auch die außerhalb des Auges angeordnete Empfangseinrichtung entsprechend ausgebildete Antennen in Form von Spulen (Ringspulen).

Es ist eine kontinuierliche Messung des Augeninnendruckes über mehrere Stufen möglich, ohne daß eine kontinuierliche Datenabfrage nach außen erforderlich ist. Durch den in der aktiven Telemetrie integrierten Datalogger können Daten gespeichert werden und zeitlich begrenzt, beispielsweise jeweils in der Woche einmal, abgefragt werden. Die Kalibrierung der implantierten Sensoren ist ohne externes Gerät durch Selbstkalibrierung möglich. Zur Kalibrierung braucht keine Brille getragen zu werden. Man erreicht eine Verringerung der eingespeisten Energie bei aktiver Telemetrie. Die Störempfindlichkeit der Messung wird durch den monolithisch integrierten Aufbau verringert. Die Meßeinrichtung ist EMV-verträglicher. Durch eine bevorzugt oberflächenmikromechanische Lösung erreicht man eine geringe Bruchempfindlichkeit der Sensoren. Ferner können oberflächenmikromechanische Sensoren in den für die Implantation erforderlichen Abmessungen hergestellt werden. Durch den monolithisch integrierten Aufbau können Siliciumchips so gedünnt werden, daß sie in das Augenimplantat, insbesondere eine Intraokularlinse, passen. Die Energieversorgung kann durch die induktive Energieübertragung von außen erfolgen, so daß eine Batterie nicht erforderlich ist.

Anhand der Figuren wird an einem Ausführungsbeispiel die Erfindung noch näher erläutert. Es zeigt:

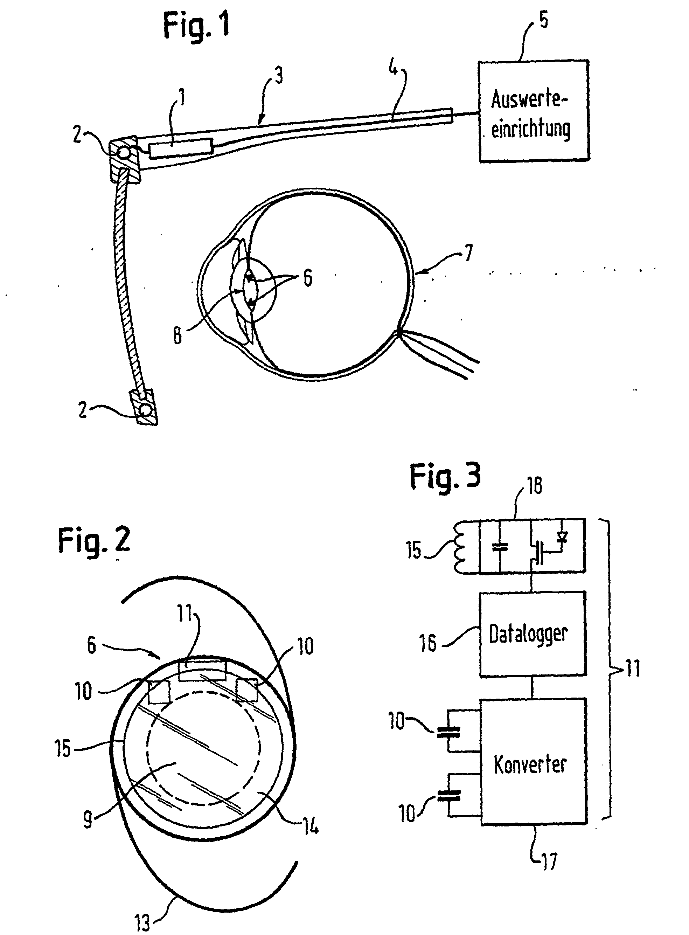

- Fig. 1:: in schematischer Seitenansicht ein Ausführungsbeispiel der Erfindung;
- Fig. 2: ein als Linse ausgebildetes Implantat, welches die bei der Erfindung zum Einsatz kommende Fernmeßeinrichtung enthält; und
- Fig. 3: ein Blockschaltbild der implantierten Fernmeßeinrichtung.

Bei dem in den Figuren dargestellten Ausführungsbeispiel ist eine Fernmeßeinrichtung 6 mittels eines als Intraokularlinse 8 ausgebildeten Augenimplantats in das Auge eingesetzt. Die Fernmeßeinrichtung 6, welche Drucksensoren 10 und sensorseitige Telemetriekomponenten, insbesondere eine Telemetrieelektronik 11 aufweist, ist im Bereich eines Haptikrandes 14, welcher einen optischen Teil 9 der Intraokularlinse umgibt, angeordnet. Die Drucksensoren 10 können in Oberflächenmikromechanik ausgebildet sein. In die Telemetrieelektronik 11 ist ein Datalogger 16 zur Speicherung der von den Drucksensoren 10 empfangenen Meßwerte angeordnet. Ferner ist die Telemetrieelektronik mit einer als Sende- und Empfangsantenne arbeitenden Spule 15 verbunden. Die im Datalogger 16 gespeicherten Meßdaten können bedarfsweise, z.B. durch Ansteuern eines elektronischen Schalters in einer mit der Spule 15 verbundenen Sendeschaltung 18 abgerufen und gesendet werden. Die Telemetrieelektronik 11 sowie die Drucksensoren können monolithisch in einen Siliciumchip integriert sein, der so dünn ausgebildet ist, daß er in den Haptikrand 14 der Linse paßt. In bevorzugter Weise ist die Linse aus Silikonmaterial hergestellt. Der Haptikrand 14 besitzt eine Breite von ca. 1 mm. Der Linsendurchmesser kann zwischen 6,5 und 7 mm variieren. Mittels Haptikfäden 13 läßt sich die Intraokularlinse beispielsweise im Kapselsack des Auges fixieren. Die Drucksensoren 10 (Sensorkomponenten) und die Telemetrieelektronik (Lesestation) mit Transponderelektronik und der als Spule 15 ausgebildeten Mikroantenne befinden sich in dem Haptikrand 14 außerhalb der optischen Zone 9 der Intraokularlinse. Die Dicke des Linsenkörpers kann je nach Brechkraft 1 bis 2 mm aufweisen.

Für das Implantatmaterial kommt beispeilsweise ein Polydiorganosiloxan, in bevorzugter Weise Polydimethylsiloxan wegen seiner guten Bioverträglichkeit und Verformbarkeit zum Einsatz. Das Implantat kann somit im gefalteten oder auch gerolltem Zustand durch einen kleinen Operationsschnitt implantiert werden. Auch für die Verkapselung der Mikrokomponenten der Fernmeßeinrichtung kann ein Polydiorganosiloxan, insbesondere Polydimethylsiloxan, verwendet werden.

Die von den Drucksensoren 10 in Abhängigkeit vom gemessenen Augeninnendruck erzeugten Meßsignale werden von der Telemetrie- und Transponderelektronik in drahtlos übertragbare Informationen in einem Konverter 17 gewandelt und mittels der als Spule 15 ausgebildeten Mikroantenne ausgesendet und außerhalb des Auges von einer Empfangseinrichtung 1 über eine ebenfalls als Spule 2 ausgebildeten Antenne empfangen. Die Empfangseinrichtung 1 und die Spule 2 können in einem Brillengestell 3 angeordnet sein. Es eignen sich jedoch auch andere Fixierungsmittel, beispielsweise eine bequeme Augenbinde. Die Empfangseinrichtung 1 ist über ein Kabel 4 mit einer Auswerteeinrichtung 5 in einem Basisgerät verbunden. Dieses Basisgerät kann neben der Auswerte- und Schnittstellenelektronik auch eine Batterie zur Energieversorgung aufweisen. Es ist jedoch auch möglich, die Energieversorgung über ein Netzkabel zu gewährleisten. Die Auswerteeinrichtung 5 wandelt die empfangenen Informationen in Daten um, welche aufgezeichnet werden können. Hierzu kann eine stationäre Datenauswerteeinrichtung vorgesehen sein für eine Offline-Datenaufbereitung, -speicherung, -analyse und -visualisierung auf PC-Basis.

Die Energieversorgung der im Auge implantierten Fernmeßeinrichtung 6 kann auf induktivem Wege über die beiden beim Sendeempfangsbetrieb als Antennen wirkenden Spulen 2 und 15 erfolgen.

## Patentansprüche

1. Vorrichtung zur Messung eines Augeninnendrucks mit einer in ein Auge (7) implantierbaren Fernmeßeinrichtung (6), welche einen Drucksensor (10), eine die Sensorsignale in drahtlos übertragbare Informationen wandelnde Einrichtung (11) und eine Sendeeinrichtung (15) enthält, einer außerhalb des Auges angeordneten Empfangseinrichtung (1, 2), welche die von der Sendeeinrichtung gesendeten Informationen empfängt, und einer Auswerteeinrichtung (5), welche die empfangenen Informationen in Daten des Augeninnendrucks wandelt, die aufgezeichnet werden können,
**dadurch gekennzeichnet,**
**daß** die in das Auge implantierbare Fernmeßeinrichtung (6) einen Datalogger (16) aufweist, in welchem die vom Drucksensor (10) kontinuierlich gelieferten Meßdaten speicherbar und aus welchem die Meßdaten beim Sende-Empfangsbetrieb zeitlich begrenzt abfragbar sind.

2. Vorrichtung nach Anspruch 1, **dadurch gekennzeichnet, daß** die insbesondere aktive Fernmeßeinrichtung (6) in oder an einem Implantat angeordnet ist.

3. Vorrichtung nach Anspruch 1 oder 2, **dadurch gekennzeichnet, daß** das Implantat als Intraokularlinse (8) ausgebildet ist.

4. Vorrichtung nach einem der Ansprüche 1 bis 3, **dadurch gekennzeichnet, daß** die Fernmeßeinrichtung (6) an einem außerhalb des optischen Linsenteils (9) liegenden Linsenteil (14) angeordnet ist.

5. Vorrichtung nach einem der Ansprüche 1 bis 4, **dadurch gekennzeichnet, daß** der Drucksensor bzw. die Drucksensoren (10) in Oberflächenmikromechanik ausgebildet sind.

6. Vorrichtung nach einem der Ansprüche 1 bis 5, **dadurch gekennzeichnet, daß** zur Verkapselung der die Fernmeßeinrichtung (6) bildenden Mikrokomponenten als Material ein Polydiorganosiloxan, insbesondere Polydimethylsiloxan, verwendet wird.

7. Vorrichtung nach einem der Ansprüche 1 bis 6, **dadurch gekennzeichnet, daß** das Implantat, insbesondere die Intraokularlinse, aus eime Polydiorganosiloxan, insbesondere Polydimethylsiloxan, gebildet ist.

8. Vorrichtung nach einem der Ansprüche 1 bis 7, dadurch gekennzeichned, daß die Komponenten der Fernmeßeinrichtung, nämlich der bzw. die Drucksensor(en), die zugehörige Signalverarbeitung, der Datalogger und die telemetrischen Komponenten, wie Spule und Kondensatoren voll monolithisch integriert sind.

## Claims

1. Apparatus for measuring an intra-ocular pressure comprising a remote measuring device (6) which is implantable in an eye (7) and which includes a pressure sensor (10), a device (11) for converting the sensor signals into wirelessly transmissible information and a transmitting device (15), a receiving device (1, 2) which is arranged outside the eye and which receives the information sent by the transmitting device, and an evaluation device (5) which converts the received information into data in respect of the intra-ocular pressure, which can be recorded,
**characterised in that**
the remote measuring device (6) which is implantable in the eye has a data logger (16) in which the measurement data continuously supplied by the pressure sensor (10) can be stored and from which the measurement data can be interrogated limitedly in respect of time in the transmitting-receiving mode.

2. Apparatus according to claim 1 **characterised in that** the in particular active remote measuring device (6) is arranged in or on an implant.

3. Apparatus according to claim 1 or claim 2 **characterised in that** the implant is in the form of an intra-ocular lens (8).

4. Apparatus according to one of claims 1 to 3 **characterised in that** the remote measuring device (6) is arranged on a lens portion (14) which is outside the optical lens portion (9).

5. Apparatus according to one of claims 1 to 4 **characterised in that** the pressure sensor or sensors (10) are designed using surface micromechanics.

6. Apparatus according to one of claims 1 to 5 **characterised in that** a polydiorganosiloxane, in particular polydimethylsiloxane, is used as a material for encapsulation of the microcomponents forming the remote measuring device (6).

7. Apparatus according to one of claims 1 to 6 **characterised in that** the implant, in particular the intra-ocular lens, is formed from a polydiorganosiloxane, in particular polydimethylsiloxane.

8. Apparatus according to one of claims 1 to 7 **characterised in that** the components of the remote measuring device, namely the pressure sensor or sensors, the associated signal processing means, the data logger, and the telemetric components such as coil and capacitors, are fully monolithically integrated.

## Revendications

1. Dispositif de mesure d'une tension intra-oculaire avec un dispositif de télémesure (6) implantable dans un oeil (7), qui comporte un capteur de pression (10), un dispositif (11) convertissant les signaux du capteur en informations transmissibles sans fil, et un dispositif d'émission (15), avec un dispositif de réception (1, 2) disposé à l'extérieur de l'oeil, qui reçoit les informations émises par le dispositif d'émission, et avec un dispositif d'analyse (5), qui convertit les informations reçues en données de la tension intra-oculaire, qui peuvent être enregistrées,
**caractérisé en ce que** le dispositif de télémesure (6) implantable dans l'oeil présente un datalogger (16), dans lequel les données de mesure fournies en continu par le capteur de pression (10) sont mémorisables, et à partir duquel les données de mesure peuvent être interrogées de façon limitée dans le temps en mode émission-réception.

2. Dispositif suivant la revendication 1, **caractérisé en ce que** le dispositif de télémesure (6) en particulier actif est disposé dans ou sur un implant.

3. Dispositif suivant l'une des revendications 1 et 2, **caractérisé en ce que** l'implant est réalisé sous forme de lentille intra-oculaire (8).

4. Dispositif suivant l'une des revendications 1 à 3, **caractérisé en ce que** le dispositif de télémesure (6) est disposé sur une partie de lentille (14) située à l'extérieur de la partie de lentille optique (9).

5. Dispositif suivant l'une des revendications 1 à 4, **caractérisé en ce que** le capteur de pression et/ou les capteurs de pression (10) sont réalisés dans la micromécanique de surface.

6. Dispositif suivant l'une des revendications 1 à 5, **caractérisé en ce qu'**un polydiorganosiloxane, en particulier du polydiméthylsiloxane, est utilisé comme matériau pour le blindage des micro-composants formant le dispositif de télémesure (6).

7. Dispositif suivant l'une des revendications 1 à 6, **caractérisé en ce que** l'implant, en particulier la lentille intra-oculaire, est formé d'un polydiorganosiloxane, en particulier de polydiméthyl-siloxane.

8. Dispositif suivant l'une des revendications 1 à 7, **caractérisé en ce que** les composants du dispositif de télémesure, à savoir le ou les capteur(s) de pression, le traitement des signaux correspondant, le datalogger et les composants télémétriques, tels que bobine et condensateurs, sont totalement intégrés de façon monolithique.
